# EUROPÄISCHE PATENTSCHRIFT

(11) **EP 1 329 430 B1**
(45) Veröffentlichungstag und Bekanntmachung des Hinweises auf die Patenterteilung: **27.03.2013**
(21) Anmeldenummer: 03008836.3
(22) Anmeldetag: 31.07.1995
(51) Int. Cl.: C03C 10/00, C03C 3/085, A61K 6/06

(54) **Alkali-Zink-Silicat-Glaskeramiken und -Gläser**
Alkali zinc silicate glass-ceramics and glasses
Vitrocéramiques et verres de silicate de zinc et alcali

(30) Priorität: 01.08.1994 DE 4428839
(43) Veröffentlichungstag der Anmeldung: 23.07.2003
(62) Teilanmeldung aus: 95250187.2
(73) Patentinhaber: Ivoclar Vivadent AG, 9494 Schaan (LI)
(72) Erfinder: Frank, Martin, FL-9494 Schaan (LI); Wegner, Susanne, CH-8422 Pfungen (CH); Rheinberger, Volker, FL-9490 Vaduz (LI); Höland, Wolfram, FL-9494 Schaan (LI)
(74) Vertreter: UEXKÜLL & STOLBERG

(56) Entgegenhaltungen:
- EP-A- 0 622 342
- DE-A- 2 034 393
- GB-A- 1 022 681
- US-A- 3 907 577
- US-A- 3 954 487
- US-A- 5 281 563
- DATABASE WPI Section Ch, Week 198112 Derwent Publications Ltd., London, GB; Class D21, AN 1981-20178D XP002265318 & JP 56 007708 A ( HOYA GLASS WORKS), 27. Januar 1981 (1981-01-27)
- DATABASE WPI Week 19864, Derwent Publications Ltd., London, GB; AN 1986-026732 -& SU 1 165 651 A1 (KHARKOV POLY) 07 Juli 1985

## Beschreibung

Die Erfindung betrifft die Verwendung von Alkali-Zink-Sillicat-Gläsern, die sich aufgrund ihrer vorteilhaften Eigenschaften, wie im Bereich von 8,0 bis 18,7 x 10⁻⁶ K⁻¹ einstellbaren linearen thermischen Ausdehnungskoeffizienten und niedrigen Verarbeitungstemperaturen als Dentalmaterial eignen, sowie geformte Dentalprodukte, die einen Gehalt an solchen Gläsern aufweisen.

In der Zahnheilkunde werden metallische Dentalrestaurationen üblicherweise mit keramischen Schichten verblendet, um das Aussehen der Restauration an das der natürlichen Zähne anzugleichen. Solche verblendeten Restaurationen werden auch als Verblendkeramiken oder Metallkeramiken bezeichnet. Um nun Spannungen zwischen dem Metallgerüst und der Keramikschicht zu vermeiden, ist es erforderlich, daß die Wärmeausdehnungskoeffizienten der keramischen Werkstoffe an die des Metalls angepaßt sind.

Es ist bekannt, daß leucithaltige Glaskeramiken sehr hohe lineare thermische Ausdehnungskoeffizienten besitzen. Diese sind auf den Gehalt an Leucit zurückzuführen, welches durch gesteuerte Kristallisation aus einem entsprechenden Ausgangsglas gebildet wird.

Damit eine Dentalglaskeramik zum Verblenden der gesamten Bandbreite von verwendeten Dentalmetallen und -legierungen, wie z.B. Titan bis hin zu Legierungen mit hohem Goldgehalt, eingesetzt werden kann, ist es erforderlich, daß ihr Ausdehnungskoeffizient in einem breiten Bereich einstellbar ist. Sofern die Dentalglaskeramik zudem auch noch als Korrekturmaterial für Aufbrennkeramiken Verwendung finden soll, so sind weiter insbesondere tiefe Sintertemperaturen von weniger als 880°C und geeignete optische Eigenschaften, wie hohe Transluzenz, sehr wünschenswert.

Bekannte Glaskeramiken und Gläser erfüllen die Forderung nach in einem weiten Bereich einstellbaren thermischen Ausdehnungskoeffizienten und einer niedrigen Verarbeitungstemperatur häufig nicht. Zudem weisen die bekannten Dentalwerkstoffe in vielen Fällen physiologisch nicht völlig unbedenkliche Komponenten, wie z.B. Sb₂O₃, auf oder es ist zwingend erforderlich, ihnen B₂O₃ zuzusetzen, um die für Dentalwerkstoffe gewünschten Eigenschaften zu erzielen. Nach Untersuchungen der Erfinder an Grundgläsern des Systems SiO₂-Al₂O₃-Na₂O-K₂O. führen geringe B₂O₃-Zusätze von ca. 3 Gew.-% zu einer unannehmbaren Verschlechterung der chemischen Beständigkeit und hohe B₂O₃-Gehalte von ca. 12 Gew.-% zu einem zu geringen Ausdehnungskoeffizienten.

Dentalkeramische Werkstoffe mit Gehalt an B₂O₃ sind z.B. aus der DE-OS 39 11 460 und der DE-PS 41 38 875 bekannt. Diese Werkstoffe besitzen relativ niedrige Verarbeitungstemperaturen und können zur Verblendung von Dentallegierungen eingesetzt werden. Ihr Wärmeausdehnungskoeffizient kann jedoch lediglich im Bereich von ungefähr 13 bis 14 × 10⁻⁶ K⁻¹ eingestellt werden. Weiter enthalten die Werkstoffe zwingend Sb₂O₃, hingegen kein ZnO und kein ZrO₂.

Ein keramischer Werkstoff zum Verblenden von metallischem Zahnersatz ist ebenfalls aus der DE-OS 40 31 168 bekannt. Auch wenn der Ausdehnungskoeffizient dieses Werkstoffes in einem Bereich von 8 bis 17,5 × 10⁻⁶ K⁻¹ einstellbar sein soll, so enthält der Werkstoff dennoch 0,7 bis 2,5 Gew.-% B₂O₃. Weiter ist in dem Werkstoff kein ZnO und kein ZrO₂ vorhanden.

Porzellanmassen mit einem hohen Gehalt an B₂O₃ von 7 bis 33 Gew.% und Verarbeitungstemperaturen im Bereich von 800°C sind aus der US-PS 5 176 747 bekannt. Diese Massen können als Dentalporzellan zur Verblendung von Titan oder Titan-Legierungen eingesetzt werden. Das verwendete B₂O₃ dient dabei sowohl zur Herabsetzung der Verarbeitungstemperatur als auch zur Herabsetzung des thermischen Ausdehnungskoeffizienten. Darüber hinaus wird dem B₂O₃ auch ein Einfluß auf die Bindungsstärke zwischen Metallsubstrat und Keramik beigemessen. Ähnliche keramische Materialien mit einem Gehalt von 8 bis 17 Gew.-% B₂O₃ werden in der EP-A-468 435 beschrieben. Diese Materialien enthalten kein ZnO und können ebenfalls zur Verblendung von Dentalrestaurationen, wie Kronen, Brücken oder Prothesenteilen eingesetzt werden, welche aus Titan oder Titan-Legierungen hergestellt sind.

Weiter sind aus der EP-A-0 155 564 leucithaltige Glaskeramiken bekannt, die jedoch B₂O₃ und physiologisch nicht unbedenkliches Sb₂O₃ enthalten.

DE 2 034 393 beschreibt ein Verfahren zur Verfestigung eines Glasartikels durch Ionenaustausch.

SU 1165651 A beschreibt Zusammensetzungen zur Erzeugung einer weißen Glasur auf Fliesen.

Der Erfindung liegt nunmehr die Aufgabe zugrunde, Gläser zu schaffen, die bei niedrigen Temperaturen durch Sinterung verarbeitbar sind, einen im Bereich von insbesondere 8,0 bis 18,7 × 10⁻⁶ K⁻¹ einstellbaren linearen thermischen Ausdehnungskoeffizienten besitzen und gleichzeitig vorteilhafte optische Eigenschaften, wie hohe Transluzenz und Opalenszenz, sowie eine ausgezeichnete chemische Beständigkeit haben und dabei ohne Zusatz von B₂O₃ und/oder physiologisch nicht völlig unbedenkliche Komponenten erzeugt werden können und demgemäß sich in vorteilhafter Weise zur Anwendung in der Dentaltechnik eignen.

Diese Aufgabe wird durch die Verwendung des Alkali-Zink-Silicat-Glases nach den Ansprüchen 1 bis 7 gelöst.

Gegenstand der vorliegenden Erfindung ist ebenfalls ein geformtes Dentalprodukt mit Gehalt an dem Glas.

Das erfindungsgemäße Alkali-Zink-Silicat-Glas ist dadurch gekennzeichnet, daß es die folgenden Komponenten enthält:

| Komponente | Gew.-% | | |
|---|---|---|---|
| SiO₂ | 52,0 | bis | 63,5 |
| Me(III)₂O₃ | 8,5 | bis | 13,0 |
| K₂O | 0 | bis | 20,5 |
| Na₂O | 6,0 | bis | 20,0 |
| Li₂O | 0 | bis | 5,0 |
| ZnO | 3,6 | bis | 8,0 |
| Me(II)O | 2,5 | bis | 6,5 |
| TiO₂ + ZrO₂ | 0,5 | bis | 6,0 |
| SnO₂ | 0 | bis | 9,5 |
| P₂O₅ | 0 | bis | 4,0 |
| F | 0 | bis | 2,0 |
| CeO₂ | 0 | bis | 3,0 |

wobei
a) die angegebene Menge Me(III)₂O₃ aus 0 bis 13 Gew.-% Al₂O₃ und 0 bis 9,5 Gew.-% La₂O₃; und
b) die angegebene Menge Me(II)O aus 0.5 bis 3,5 Gew.-% CaO, 0 bis 4,5 Gew.-% BaO und 0 bis 5,0 Gew.-% MgO
gebildet ist.

Vorzugsweise besteht das Glas im wesentlichen aus den zuvor genannten Komponenten.

Für einige der Komponenten des Glases existieren bevorzugte Mengenbereiche. Diese können unabhängig voneinander gewählt werden und sind wie folgt:

| Komponente | | Gew.-% | | |
|---|---|---|---|---|
| SiO₂ | | 52,0 | bis | 61,0 |
| Al₂O₃ | | 8,5 | bis | 11,0 |
| La₂O₃ | | 0 | bis | 2,0 |
| K₂O | | 0 | bis | 15,0 |
| Na₂O | | 6,0 | bis | 15,0 |
| Li₂O | | 0 | bis | 4,0 |
| ZnO | 3,6 bis 7,0, insbesondere | 4,0 | bis | 7,0 |
| CaO | | 0,5 | bis | 3,5 |
| BaO | | 1,0 | bis | 4,5 |
| TiO₂ | | 0 | bis | 2,8 |
| ZrO₂ | | 0,5 | bis | 5,0 |
| P₂O₅ | | 0 | bis | 2,0 |

Es ist ganz besonders bevorzugt, daß das erfindungsgemäße Glas im wesentlichen frei von B₂O₃, Antimon- und/oder Bleiverbindungen ist.

Zur Herstellung des erfindungsgemäßen Glases wird vorzugsweise so vorgegangen, daß geeignete Ausgangsmaterialien, wie z.B. Carbonate, Oxide und Fluoride, bei einer Temperatur im Bereich von 1350 bis 1650°C, vorzugsweise 1400 bis 1600°C, über einen Zeitraum von 30 Minuten bis 4 Stunden, vorzugsweise eine Stunde bis 2,5 Stunden, unter Bildung einer homogenen Schmelze erschmolzen werden. Das erschmolzene Glas wird anschließend in Wasser abgeschreckt, d.h. gefrittet, und das erhaltene Glasgranulat wird nach Trocknen aufgemahlen.

Zur Herstellung einer Glaskeramik wird insbesondere so vorgegangen, daß das erhaltene Granulat des erfindungsgemäßen Glases einer thermischen Behandlung bei einer Temperatur im Bereich von 600 bis 900°C für eine Dauer von 30 Minuten bis 5 Stunden, vorzugsweise 30 Minuten bis 2 Stunden, unterzogen wird. Vorzugsweise wird das hierbei eingesetzte Glas vor der Wärmebehandlung zu einem Pulver mit einer Korngröße von weniger als 90 µm gemahlen und gesiebt. Die thermische Behandlung kann auch durch die im Rahmen der Herstellung von geformten Dentalprodukten aus dem erfindungsgemäßen Glas erforderlichen Wärmebehandlungen, wie Sinterungsvorgänge, bewirkt werden.

Mittels rasterelektronenmikroskopischer Untersuchungen wurde festgestellt, daß die erfindungsgemäßen Gläser frei von Kristallen sind oder nur sehr vereinzelt Kristalle aufweisen. Hingegen enthalten die Glaskeramiken Kristalle, insbesondere Leucitkristalle, welche durch die gesteuerte Oberflächenkristallisation im Rahmen der thermischen Behandlung gebildet wurden. Vorzugsweise bilden die Leucitkristalle die Hauptkristallphase in den Glaskeramiken, und die mittlere Größe der Leucitkristalle beträgt vorzugsweise weniger als 5 µm bezogen auf die Anzahl der Kristalle.

Zusätzlich zu Leucitkristallen können je nach chemischer zusammensetzung des eingesetzten Glases weitere Kristallphasen gebildet werden. Neben den verschiedenen Kristallphasen können auch mikroheterogene Entmischungsbereiche, d.h. verschiedene Glasphasen, vorliegen. Diese Bereiche sind im Rasterelektronenmikroskop als kleine mikroheterogene Tropfenglasphasen mit einer Größe von ca. 40 bis 250 nm erkennbar. Das Vorliegen dieser Tropfenglasphasen oder von Kristallen beeinflußt die optischen Eigenschaften, wie z.B. Opaleszenz und Transluzenz, der erfindungsgemäßen Gläser.

Der lineare thermische Ausdehnungskoeffizient der erfindungsgemäßen Gläser kann vorzugsweise im Bereich von 8,0 bis 18,7 × 10⁻⁶ K⁻¹, gemessen im Temperaturbereich von 100 bis 400°C, eingestellt werden. Es ist überraschend, daß trotz des hohen Gehalts von 52,5 bis 63,5 Gew.-% SiO₂ und ohne Zusatz von B₂O₃ den erfindungsgemäßen Gläsern sowohl hohe als auch niedrige Ausdehnungskoeffizienten verliehen werden können. Nach dem Stand der Technik ist zur Einstellung von niedrigen Ausdehnungskoeffizienten hingegen ein Zusatz von B₂O₃ in den meisten Fällen zwingend erforderlich.

Durch die Verwendung von einwertigen Netzwerkwandlerionen, wie Kalium, Natrium und Lithium, und durch Einsatz von Fluor konnte die Verarbeitungstemperatur gesenkt werden. So können die erfindungsgemäßen Gläser in Pulverform bei Temperaturen von vorzugsweise 640 bis 850°C zusammengesintert und damit verarbeitet werden.

Der hohe Gehalt an ZnO in den erfindungsgemäßen Gläsern trägt wesentlich zur guten chemischen Beständigkeit bei und reduziert im Vergleich zu ZrO₂ deren Viskosität. Darüber hinaus zeichnet sich ZnO durch eine ausgezeichnete physiologische Verträglichkeit aus.

Neben den zuvor erwähnten Komponenten können die erfindungsgemäßen Gläser außerdem Zusatzstoffe, wie z.B. Farbstoffe, insbesondere Farbpigmente, Oxide der 3d-Elemente oder Metallkolloide, oder Fluoreszenzstoffe, insbesondere mit d- und f-Elementen dotiertes Ytterbium-Silicat, enthalten. Weitere geeignete Zusatzstoffe zur Veränderung von z.B. den optischen und/oder thermischen Eigenschaften der erfindungsgemäßen Gläser sind weitere Gläser, Keramiken, Glaskeramiken, Trübungsstoffe und/oder Stabilisatoren.

Die erfindungsgemäßen Gläser können entweder allein als Dentalmaterial oder als Bestandteil von Dentalmaterialien, wie z.B. Dentalkeramikpulvern, eingesetzt werden. Hierzu wird das erfindungsgemäße Glas vorzugsweise in Form eines Pulvers mit einer Teilchengröße von insbesondere weniger als 90 µm eingesetzt. Dieses Glaspulver eignet sich dabei in besonders vorteilhafter Weise als Korrekturmaterial für metallkeramische oder vollkeramische Dentalsuprastrukturen, wie z.B. eine Teilkrone, eine Krone oder eine Brücke. Für diesen Zweck wird das Pulver auf die gewünschten Stellen der Dentalsuprastruktur aufgebracht und bei Temperaturen von 640 bis 850°C in einem Vakuumbrennofen zusammengesintert. Dabei können die Eigenschaften des Pulvers, wie z.B. thermischer Ausdehnungskoeffizient und optische Eigenschaften an die des jeweils aufgesinterten Basismaterials angepaßt werden.

Eine Glaskeramik kann auch für sich als Schicht- oder Verblendmaterial für vollkeramische, metallische oder in Form von Legierungen vorliegende Dentalsuprastrukturen verwendet werden. Hierzu wird die gepulverte Glaskeramik mit Wasser vermischt und auf das metallische oder vollkeramische Substrat aufgebracht. Nach Formung der gewünschten Dentalrestauration, wie z.B. Brücke oder Krone, wird diese bei Temperaturen von 640 bis 850°C zu dem fertigen, geformten Dentalprodukt dichtgesintert. Dabei ist es von besonderem Vorteil, daß der thermische Ausdehnungskoeffizient der Glaskeramik innerhalb eines breiten Bereichs variiert werden kann, und zwar auf 8,0 × 10⁻⁶ K⁻¹ für ein Titansubstrat und ungefähr 16,0 × 10⁻⁶ K⁻¹ für ein Substrat aus Gold oder Legierung mit hohem Goldgehalt eingestellt und damit an den Ausdehnungskoeffizienten des verwendeten Substrats angepaßt werden kann.

Es ist besonders überraschend, daß die erfindungsgemäßen Gläser eine Kombination von niedriger Verarbeitungstemperatur, in einem breiten Bereich einstellbaren thermischen Ausdehnungskoeffizienten und sehr gute chemische Beständigkeit aufweisen.

Als erfindungsgemäße geformte Dentalprodukte, die einen Gehalt an dem erfindungsgemäßen Glas aufweisen, kommen Kronen, Teilkronen und Brücken in Frage.

Die Erfindung wird nachstehend anhand von Beispielen näher erläutert.

### Beispiele

### Beispiele 1 bis 21

Es wurden insgesamt 21 verschiedene Glaskeramiken und 21 verschiedene erfindungsgemäße Gläser hergestellt. Sie hatten die in der Tabelle I angegebenen chemischen Zusammensetzungen.

Für einige der Glaskeramiken und Gläser sind in Tabelle II ausgewählte Eigenschaften angegeben, die an Probekörpern aus dem jeweiligen Glas oder der jeweiligen Glaskeramik bestimmt worden sind. Weiter finden sich in Tabelle II unter "Temperaturbehandlung" Angaben zu dem jeweils eingesetzten Ausgangsmaterial für die Probekörper sowie Angaben zu einer etwaigen Wärmebehandlung des Ausgangsmaterials. Bei allen Ausgangsmaterialien, für die keine Wärmebehandlung angegeben ist, handelte es sich um erfindungsgemäße Gläser. Die Ausgangsmaterialien mit angegebener Wärmebehandlung waren Glaskeramiken. Es ist jedoch zu beachten, daß im Falle des nicht-wärmebehandelten Glases Nr. 12 eine Umwandlung zu einer entsprechenden Glaskeramik eintrat, wenn dieses im in Tabelle II angegebenen Temperaturbereich zu Probekörpern gesintert wurde.

Die Tabelle II zeigt weiter, daß eine Glaskeramik in der Regel einen höheren Ausdehnungskoeffizienten als ein Glas entsprechender chemischer Zusammensetzung hat.

Die Beispiele verdeutlichen wie durch Veränderung der chemischen Zusammensetzung und durch etwaige Wärmebehandlung Glaskeramiken und Gläser mit unterschiedlichen Eigenschaften erhalten werden können.

**Tabelle I**

| | SiO₂ | Al₂O₃ | La₂O₃ | K₂O | Na₂O | Li₂O | CaO | BaO | MgO | ZnO | TiO₂ | ZrO₂ | SnO₂ | P₂O₅ | F | CeO₂ |
|---|---|---|---|---|---|---|---|---|---|---|---|---|---|---|---|---|
| 1 | 57,0 | --- | 9,4 | 13,8 | 6,4 | 1,7 | 1,8 | 4,0 | --- | 4,1 | --- | 1,0 | --- | --- | 0,8 | --- |
| 2 * | 55,4 | 2,9 | 6,3 | 20,2 | 1,8 | 1,6 | 1,8 | 4,0 | --- | 4,0 | --- | 1,1 | --- | --- | 0,9 | --- |
| 3 | 53,7 | 2,9 | 6,3 | 13,5 | 6,3 | 1,6 | 1,8 | 4,0 | --- | 4,0 | --- | 1,0 | --- | 4,0 | 0,9 | --- |
| 4 | 58,3 | 9,7 | --- | 5,6 | 12,4 | 1,7 | 1,9 | 4,2 | --- | 4,2 | --- | 1,1 | --- | --- | 0,9 | --- |
| 5 * | 54,5 | 10,0 | --- | --- | 20,0 | 2,5 | | --- | 5,0 | 4,0 | 1,0 | 1,0 | --- | --- | 2,0 | --- |
| 6 | 52,3 | 10,2 | --- | 8,7 | 9,6 | 2,6 | 1,3 | 3,7 | --- | 7,4 | 2,8 | 1,4 | --- | --- | --- | --- |
| 7 | 54,7 | 10,8 | --- | 9,2 | 9,3 | 2,4 | 1,4 | 3,9 | --- | 7,8 | --- | 0,5 | --- | --- | --- | --- |
| 8 | .57,0 | 10,4 | --- | 13,8 | 6,9 | 1,7 | 1,6 | 4,1 | --- | 3,1 | --- | 0,6 | --- | --- | 0,8 | --- |
| 9 | 56.6 | 9,6 | --- | 13,1 | 9,2 | --- | 1,9 | 4,1 | --- | 3,1 | 0,5 | 1,0 | --- | --- | 0,9 | --- |
| 10 | 60,3 | 10,0 | --- | 9,1 | 7,3 | 3,5 | 3,2 | 1,1 | --- | 4,4 | --- | 1,1 | --- | --- | | --- |
| 11 | 57,6 | 9,5 | --- | 10,1 | 7,0 | 4,2 | 1,9 | 4,1 | --- | 4,1 | 0,5 | 1,0 | --- | --- | --- | --- |
| 12 | 57,4 | 8,5 | 1,0 | 11,0 | 8,4 | 1,7 | 1,9 | 4,1 | --- | 4,1 | --- | 1,0 | --- | --- | 0,9 | --- |
| 13 | 57,5 | 9,6 | --- | 9,7 | 9,4 | 1,7 | 1,9 | 4,1 | --- | 4,2 | --- | 1,0 | --- | --- | 0,9 | --- |
| 14 | 56,0 | 8,4 | 1,0 | 10,9 | 8,3 | 1,6 | 1,8 | 4,1 | --- | 4,1 | --- | 1,4 | --- | 1,5 | 0,9 | --- |
| 15 | 56,5 | 9,4 | --- | 13,8 | 6,4 | 1,7 | 1,8 | 4,4 | --- | 4,1 | --- | 1,0 | --- | --- | 0,9 | --- |
| 16 | 63,3 | 10,5 | --- | --- | 9,2 | 4,9 | 0,9 | --- | 1,8 | 7,3 | --- | 1,1 | --- | --- | 1,0 | --- |
| 17 | 58,8 | 9,8 | --- | --- | 10,5 | 2,7 | 0,9 | --- | 1,7 | 4,2 | --- | 1,1 | 9,4 | --- | 0,9 | --- |
| 18 | 58,5 | 11,3 | --- | --- | 8,6 | 2,9 | 0,9 | --- | 1,7 | 4,2 | --- | 6,0 | 5,0 | --- | 0,9 | --- |
| 19 | 58,3 | 13,0 | --- | --- | 8,6 | 2,9 | 0,9 | --- | 1,7 | 4,2 | --- | 5,5 | 4,0 | --- | 0,9 | --- |
| 20 | 56,7 | 9,6 | --- | 12,8 | 9,2 | --- | 1,9 | 4,1 | --- | 3,6 | --- | 1,2 | --- | --- | 0,9 | --- |
| 21 | 56,0 | 9,4 | --- | 9,1 | 8,9 | 1,6 | 1,9 | 4,0 | --- | 4,1 | --- | 1,0 | --- | --- | 1,0 | 3,0 |

| | | | | | | | | | | | | | | | | |
|---|---|---|---|---|---|---|---|---|---|---|---|---|---|---|---|---|
| *(Vergleichsbeispeil) | | | | | | | | | | | | | | | | |

**Tabelle II**

| Beispiel Nr. | Temperaturbehandlung | Sintertemperatur oder -bereich zur Herstellung von Stäben für α-Messung | lin., therm. Ausdehnungs-koeffizient α [10⁻⁶ K⁻¹)] (100 - 400°C) | Tg [°C] | Säurebeständigkeit [%] (Masseverlust nach ISO 6872) | Optisches Verhalten |
|---|---|---|---|---|---|---|
| 1 | Glaspulver <90 µm 750°C, 2 h | 710°C/690°C | 12,1 | 461 | --- | transluzent |
| 2 * | Glaspulver <90 µm | 740°C/720°C | 11,6 | 489 | 0,05 | sehr transluzent |
| 3 | Glaspulver <90 µm | 740°C/720°C | 12,1 | 464 | 0,02 | sehr transluzent |
| 4 | Glaspulver <90 µm | 730°C/710°C | 11,2 | 453 | 0,02 | sehr transluzent |
| 6 | Glaspulver <90 µm | 700°C/720°C | 11,9 | 487 | | --- |
| 8 | Glaspulver <90 µm | 700°C/720°C | 11,5 | 460 | 0,05 0,04 | sehr transluzent |
| 8 | Glaspulver <90 µm 600°C, 60' | 700°C/720°C | 14,0 | 430 | 0,04 | transluzent |
| 9 | Glaspulver <90 µm | 760°C/780°C | 11,5 | 493 | 0,02 | sehr transluzent |
| 10 | Glaspulver <90 µm | 700°C/720°C | 11,3 | 480 | 0,01 | sehr transluzent |
| 12 | Glaspulver <90 µm | 720°C/700°C | 13,4 | 443 | 0,03 | sehr transluzent |
| 12 | Glaspulver <90 µm 750°C, 1 h | 720°C/740°C | 16,2 | 431 | 0,02 | transluzent |
| 13 | Glaspulver <90 µm | 730°C/710°C | 11,8 | 452 | 0,02 | sehr transluzent |
| 13 | Glaspulver <90 µm 750°C, 1 h | 710°C/690°C | 14,7 | 437 | 0,03 | transluzent |
| 14 | Glaspulver <90 µm | 740°C/720°C | 12,1 | 454 | 0,02 | sehr transluzent |
| 14 | Glaspulver <90 µm 750°C, 1 h | 740°C/720°C | 16,3 | 438 | 0,02 | transluzent opal |
| 14 | Fritte getempert 750°C, 1 h | 750°C | 11,7 | 449 | 0,02 | sehr transluzent, opal |
| 15 | Glaspulver <90 µm 750°C, 30' | 750°C/730°C | 18,7 | 408 | 0,05 | transluzent |
| 17 | Glaspulver <90 µm | 780°C/800°C | 9,0 | 524 | 0,008 | transluzent |

| | | | | | | |
|---|---|---|---|---|---|---|
| *(Vergleichsbeispeil) | | | | | | |

### Beispiel 22

Dieses Beispiel beschreibt die Herstellung einer Glaskeramik, die als tief schmelzende Verblendkeramik oder als Korrekturmaterial von sowohl Verblend- als auch Vollkeramiken verwendet werden kann.

Zunächst wurde ein Ausgangsglas mit der in Tabelle I für Beispiel 12 angegebenen chemischen Zusammensetzung hergestellt. Dazu wurde ein Gemenge von Oxiden, Carbonaten und Fluoriden in einem Platin/Rhodium-Tiegel bei einer Temperatur von 1550 bis 1600°C während einer Homogenisierungszeit von ungefähr 2 Stunden erschmolzen. Die Glasschmelze wurde in Wasser abgeschreckt, und die gebildete Glasfritte wurde getrocknet und auf eine Korngröße von weniger als 90 µm aufgemahlen. Anschließend wurde das erhaltene Glaspulver eine Stunde lang bei 750°C wärmebehandelt, erneut aufgemahlen und auf eine Teilchengröße von weniger als 90 µm gesiebt. Mit dem erhaltenen Glaskeramikpulver wurden Prüfkörper hergestellt und die in Tabelle II unter Nr. 12, mit Wärmebehandlung, angegebenen Eigenschaften bestimmt.

Zur Messung des linearen thermischen Ausdehnungskoeffizienten wurde aus dem Glaskeramikpulver ein stäbchenförmiger Grünkörper hergestellt, der in einem Vakuumbrennofen mit einer Aufheizrate von 60°C/min und einer Haltezeit von 1 Minute bei einer Temperatur von 720°C gebrannt wurde. Anschließend wurde ein Glanzbrand ohne Vakuum bei einer Endtemperatur von 740°C und einer Haltezeit von 1 Minute durchgeführt. Der lineare thermische Ausdehnungskoeffizient für den erhaltenen Probekörper betrug 16,2 × 10⁻⁶ K⁻¹, gemessen im Temperaturbereich von 100 bis 400°C. Damit ist der Ausdehnungskoeffizient dieser Glaskeramik an den von Legierungen mit hohem Goldgehalt angepaßt.

Auch bei der Herstellung von Plättchen anstelle von Stäben konnte die Glaskeramik bei einer sehr niedrigen Temperatur von nur 740°C zusammengesintert werden. Die Herstellung der Plättchen erfolgte in der Weise, daß aus dem Glaskeramikpulver ein Grünling in Plättchenform gebildet und dieser auf einem mit Quarzmehl bestrichenen Brenngutträger im Vakuumofen bei 740°C und einer Haltezeit von einer Minute gebrannt wurde. Die Aufheizrate des Vakuumofens betrug dabei 60°C/min.

Die Bestimmung der chemischen Beständigkeit der Glaskeramik durch Behandlung von gesinterten Plättchen mit 4%iger Essigsäure in einer Soxhlet-Apparatur gemäß ISO 6872 führte zu einem sehr geringen Masseverlust der Glaskeramik von nur 0,02%.

Durch den an Legierungen mit hohem Goldgehalt angepaßten linearen thermischen Ausdehnungskoeffizienten, die sehr gute chemische Beständigkeit und die niedrige Verarbeitungstemperatur ist diese Glaskeramik besonders zum Aufsintern auf solche Legierungen sowie als Korrekturmaterial für Verblend- und Vollkeramiken geeignet.

Zur Erzielung von linearen thermischen Ausdehnungskoeffizienten, die auf alle derzeit üblichen goldhaltigen Dentallegierungen angepaßt sind, kann die Glaskeramik vorzugsweise mit anderen gepulverten Glaskeramiken und Gläsern einer in Tabelle I angegebenen chemischen Zusammensetzung gemischt werden.

### Beispiel 23

Dieses Beispiel beschreibt die Herstellung einer Glaskeramik, die als Korrekturmaterial für Vollkeramiken und insbesondere für Metallkeramiken eingesetzt werden kann.

Zunächst wurde ein Glas mit der in Tabelle I für Beispiel 13 angegebenen Zusammensetzung entsprechend der in Beispiel 22 beschriebenen Verfahrensweise erschmolzen und aufgemahlen. Das erhaltene Pulver wurde als Pulver I bezeichnet.

Weiter wurde ein Glas mit der in Tabelle I für Beispiel 14 angegebenen Zusammensetzung ebenfalls entsprechend der Verfahrensweise gemäß Beispiel 22 erschmolzen und gefrittet. Die getrocknete Fritte wurde dann eine Stunde lang bei 750°C wärmebehandelt und schließlich in einer Achatmühle aufgemahlen und auf eine Teilchengröße von weniger als 90 µm gesiebt. Das erhaltene Pulver wurde als Pulver II bezeichnet.

Durch geeignetes Mischen dieser zwei Pulver mit dem Glaskeramikpulver gemäß Beispiel 22 konnte der Ausdehnungskoeffizient in gewünschter Weise eingestellt und damit die erhaltene Mischung als Korrekturmaterial zum Aufsintern auf Metallkeramikkronen mit sehr guten optischen Eigenschaften verwendet werden. Beispielsweise bestand eine geeignete Mischung aus 70 Gew.-% Pulver I, 15 Gew.-% Pulver II und 15 Gew.-% Pulver gemäß Beispiel 22, und diese Mischung hatte einen Ausdehnungskoeffizienten von 12,7 × 10⁻⁶ K⁻¹.

Zur Verwendung als Korrekturmaterial wurde diese Mischung auf die zu korrigierende Stelle einer Metallkeramikkrone aufgebracht, und die Krone wurde bei einer Temperatur von 640°C gebrannt, wobei ab 580°C unter Vakuum gearbeitet wurde, die Aufheizrate 60°C/min und die Haltezeit eine Minute betrug. Die fertige Krone hatte an der korrigierten Stelle ein sehr transluzentes und insbesondere im Schneidebereich leicht opaleszentes Aussehen und wirkte dadurch lebhaft.

Bei einer Temperatur von 730°C und einer Haltezeit von einer Minute konnten aus dieser Mischung Plättchen auf Quarzmehl gebrannt werden. Außerdem zeigten gemäß ISO 6872 hergestellte und geprüfte Plättchen aus dieser Mischung eine sehr gute Säurebeständigkeit von lediglich 0,02% Masseverlust.

### Beispiel 24

Dieses Beispiel beschreibt eine Glaskeramik, die als Korrekturmaterial für Verblendkeramiken und insbesondere für Vollkeramikkronen verwendet werden kann.

Zunächst wurde ein Glas mit der in Tabelle I für Beispiel 15 angegebenen chemischen Zusammensetzung gemäß der in Beispiel 22 beschriebenen Verfahrensweise erschmolzen und gemahlen. Das erhaltene Glaspulver wurde dann 30 Minuten lang bei 750°C wärmebehandelt. Die Eigenschaften der erhaltenen Glaskeramik sind in Tabelle II unter Nr. 15 angegeben.

Durch geeignetes Mischen eines Pulvers dieser Glaskeramik mit dem Pulver I gemäß Beispiel 23 konnte der Ausdehnungskoeffizient so eingestellt werden, daß die erhaltene Mischung als Korrekturmaterial zum Aufsintern auf vollkeramische Kronen verwendet werden konnte. Eine für diesen Zweck geeignete Mischung enthielt 80 Gew.-% des Glaskeramikpulvers und 20 Gew.-% Glaspulver I gemäß Beispiel 23 und hatte einen Ausdehnungskoeffizienten von 16,7 × 10⁻⁶ K⁻¹, gemessen im Bereich von 100 bis 400°C.

### Beispiel 25

Dieses Beispiel beschreibt ein erfindungsgemäßes Glas, das einen linearen thermischen Ausdehnungskoeffizienten von ca. 8,0 × 10⁶ K⁻¹, gemessen im Bereich von 100 bis 500°C, aufweist und demgemäß für Titanlegierungen als Verblendmaterial oder Korrekturmaterial verwendet werden kann. Das Glas besitzt eine Verarbeitungstemperatur, d.h. eine Sinterungstemperatur, von weniger als 880°C.

Zu seiner Herstellung wurde ein Glas mit der in Tabelle I für Beispiel 18 angegebenen chemischen Zusammensetzung gemäß der in Beispiel 22 angegebenen Verfahrensweise erschmolzen und aufgemahlen. Die Herstellung von Prüfkörpern erfolgte entsprechend Beispiel 22, wobei jedoch die Brenntemperatur zur Herstellung der stäbchenförmigen Prüfkörper für die Messung des Ausdehnungskoeffizienten beim ersten Brand 850°C betrug und der Glanzbrand bei 830°C durchgeführt wurde. Für die so hergestellten stäbchenförmigen Prüfkörper wurde ein thermischer Ausdehnungskoeffizient von 8,1 × 10⁻⁶ K⁻¹, gemessen im Temperaturbereich von 100 bis 500°C, bestimmt.

Die Brenntemperatur von Plättchen auf Quarzmehl betrug lediglich 850°C, und gemäß ISO 6872 hergestellte und untersuchte Plättchen aus dem Glas zeigten eine sehr gute Säurebeständigkeit von lediglich 0,01% Masseverlust. Außerdem besaßen aus dem Glas gebrannte Plättchen eine sehr hohe Transluzenz.

## Patentansprüche

1. Verwendung eines Alkali-Zink-Silicat-Glases, das die folgenden Komponenten enthält:
| Komponente | Gew.-% | | |
|---|---|---|---|
| SiO₂ | 52,0 | bis | 63,5 |
| Me(III)₂O₃ | 8,5 | bis | 13,0 |
| K₂O | 0 | bis | 20,5 |
| Na₂O | 6,0 | bis | 20,0 |
| Li₂O | 0 | bis | 5,0 |
| ZnO | 3,6 | bis | 8,0 |
| Me(II)O | 2,5 | bis | 6,5 |
| TiO₂ + ZrO₂ | 0,5 | bis | 6,0 |
| SnO₂ | 0 | bis | 9,5 |
| P₂O₅ | 0 | bis | 4,0 |
| F | 0 | bis | 2,0 |
| CeO₂ | 0 | bis | 3,0, |
wobei
a) die angegebene Menge Me(III)₂O₃ aus 0 bis 13 Gew.-% Al₂O₃ und 0 bis 9,5 Gew.-% La₂O₃; und
b) die angegebene Menge Me(II)O aus 0,5 bis 3,5 Gew.-% CaO, 0 bis 4,5 Gew.-% BaO und 0 bis 5,0 Gew.-% MgO
gebildet ist,
als Dentalmaterial oder Bestandteil von Dentalmaterial.

2. Verwendung nach Anspruch 1, **dadurch gekennzeichnet, dass** die Mengen von einigen Komponenten unabhängig voneinander wie folgt sind:
| Komponente | | Gew.-% | | |
|---|---|---|---|---|
| SiO₂ | | 52,0 | bis | 61,0 |
| Al₂O₃ | | 8,5 | bis | 11,0 |
| La₂O₃ | | 0 | bis | 2, 0 |
| K₂O | | 0 | bis | 15,0 |
| Na₂O | | 6,0 | bis | 15,0 |
| Li₂O | | 0 | bis | 4,0 |
| ZnO | 3,6 bis 7,0, insbesondere | 4,0 | bis | 7,0 |
| BaO | | 1,0 | bis | 4,5 |
| TiO₂ | | 0 | bis | 2,8 |
| ZrO₂ | | 0,5 | bis | 5,0 |
| P₂O₅ | | 0 | bis | 2,0 |

3. Verwendung nach Anspruch 1 oder 2, **dadurch gekennzeichnet, dass** das Glas im Wesentlichen frei von B₂O₃ ist.

4. Verwendung nach einem der Ansprüche 1 bis 3, **dadurch gekennzeichnet, dass** das Glas als Zusatzstoffe Farbstoffe, Fluoreszenzstoffe, weitere Gläser, Keramiken, Glaskeramiken, Trübungsstoffe und/oder Stabilisatoren enthält.

5. Verwendung nach einem der Ansprüche 1 bis 4, **dadurch gekennzeichnet, dass** das Glas einen linearen thermischen Ausdehnungskoeffizienten von 8,0 bis 18,7 x 10⁻⁶ K⁻¹, gemessen im Bereich von 100 bis 400°C, hat.

6. Verwendung nach einem der Ansprüche 1 bis 5, wobei das Glas als Korrekturmaterial für metallkeramische oder vollkeramische Dentalsuprastrukturen eingesetzt wird.

7. Verwendung nach Anspruch 6, wobei die Dentalsuprastruktur die Form einer Krone, einer Brücke oder einer Teilkrone hat.

8. Geformtes Dentalprodukt, welches einen Gehalt an dem Glas gemäß einem der Ansprüche 1 bis 5 aufweist und die Form einer Krone, einer Teilkrone oder einer Brücke hat.

## Claims

1. Use of an alkali-zinc-silicate glass comprising the following components:
| Component | wt.-% | | |
|---|---|---|---|
| SiO₂ | 52.0 | to | 63.5 |
| Me(III)_{z}O₃ | 8.5 | to | 13.0 |
| K₂O | 0 | to | 20.5 |
| Na₂O | 6.0 | to | 20.0 |
| Li₂O | 0 | to | 5.0 |
| ZnO | 3.6 | to | 8.0 |
| Me(II)O | 2.5 | to | 6.5 |
| TiO₂ + ZrO₂ | 0.5 | to | 6.0 |
| SnO₂ | 0 | to | 9.5 |
| P₂O₅ | 0 | to | 4.0 |
| F | 0 | to | 2.0 |
| CeO₂ | 0 | to | 3.0, |
wherein
a) the quantity of Me(III)₂O₃ given is formed from 0 to 13 wt.-% A1203 and 0 to 9.5 wt.-% La₂O₃; and
b) the quantity of Me(II)O given is formed from 0.5 to 3.5 wt.-% CaO, 0 to 4.5 wt.-% BaO und 0 to 5.0 wt.-% MgO,
as a dental material or constituent of a dental material.

2. Use according to Claim 1, **characterised in that** the quantities of some components, independently of one another, are as follows:
| Component | | wt.-% | | |
|---|---|---|---|---|
| SiO₂ | | 52.0 | to | 61.0 |
| Al₂O₃ | | 8.5 | to | 11.0 |
| La₂O₃ | | 0 | to | 2.0 |
| K₂O | | 0 | to | 15.0 |
| Na₂O | | 6.0 | to | 15.0 |
| Li₂O | | 0 | to | 4.0 |
| ZnO | 3.6 to 7.0, in particular | 4.0 | to | 7.0 |
| BaO | | 1.0 | to | 4.5 |
| TiO₂ | | 0 | to | 2.8 |
| ZrO₂ | | 0.5 | to | 5.0 |
| P₂O₅ | | 0 | to | 2.0 |

3. Use according to claim 1 or 2 **characterised in that** the glass is essentially free from B₂O₃.

4. Use according to one of claims 1 to 3 **characterised in that** the glass comprises as additives colouring agents, fluorescent agents, further glasses, ceramics, glass ceramics, opacifiers and/or stabilisers.

5. Use according to one of claims 1 to 4 **characterised in that** the glass has a linear thermal expansion coefficient of 8.0 to 18.7 x 10⁻⁶ K⁻¹, measured in the range of from 100 to 400°C.

6. Use according to one of claims 1 to 5, wherein the glass is used as correction material for metal ceramic or all-ceramic dental suprastructures.

7. Use according to claim 6, wherein the dental suprastructure has the shape of a crown, a bridge or a partial crown.

8. Moulded dental product comprising the glass according to any one of claims 1 to 5 and having the shape of a crown, a partial crown or a bridge.

## Revendications

1. Utilisation, en tant que matériau dentaire ou constituant d'un matériau dentaire, d'un verre de silicate d'alcali et de zinc qui contient les composants suivants :
| Composant | % en poids | | |
|---|---|---|---|
| SiO₂ | 52,0 | à | 63,5 |
| Me(III)₂O₃ | 8,5 | à | 13,0 |
| K₂O | 0 | à | 20,5 |
| Na₂O | 6,0 | à | 20,0 |
| Li₂O | 0 | à | 5, 0 |
| ZnO | 3, 6 | à | 8, 0 |
| Me(II)O | 2,5 | à | 6,5 |
| TiO₂ + ZrO₂ | 0,5 | à | 6,0 |
| SnO₂ | 0 | à | 9, 5 |
| P₂O₅ | 0 | à | 4 , 0 |
| F | 0 | à | 2,0 |
| CeO₂ | 0 | à | 3, 0 |
où
a) la quantité indiquée de Me(III)₂O₃ est formée de 0 à 13 % en poids d'Al₂O₃ et de 0 à 9,5 % en poids de La₂O₃ ; et
b) la quantité indiquée de Me(II)O est formée de 0,5 à 3,5 % en poids de CaO, de 0 à 4,5 % en poids de BaO et de 0 à 5,0 % en poids de MgO.

2. Utilisation selon la revendication 1, **caractérisée en ce que** les quantités de quelques composants sont les suivantes, indépendamment les uns des autres :
| Composant | | % en poids | | |
|---|---|---|---|---|
| SiO₂ | | 52,0 | à | 61,0 |
| Al₂O₃ | | 8,5 | à | 11,0 |
| La₂O₃ | | 0 | à | 2,0 |
| K₂O | | 0 | à | 15,0 |
| Na₂O | | 6,0 | à | 15,0 |
| Li₂O | | 0 | à | 4,0 |
| ZnO | 3,6 à 7,0, en particulier | 4,0 | à | 7,0 |
| BaO | | 1,0 | à | 4,5 |
| TiO₂ | | 0 | à | 2,8 |
| ZrO₂ | | 0,5 | à | 5,0 |
| P₂O₅ | | 0 | à | 2,0 |

3. Utilisation selon la revendication 1 ou 2, **caractérisée en ce que** le verre est pour l'essentiel exempt de B₂O₃.

4. Utilisation selon l'une des revendications 1 à 3, **caractérisée en ce que** le verre contient en tant qu'additifs des colorants, des agents fluorescents, d'autres verres, des céramiques, des vitrocéramiques, des agents opacifiants et/ou des stabilisants.

5. Utilisation selon l'une des revendications 1 à 4, **caractérisée en ce que** le verre présente un coefficient de dilatation thermique linéaire de 8,0 à 18,7 x 10⁻⁶ K¹, mesuré dans la plage de 100 à 400°C.

6. Utilisation selon l'une des revendications 1 à 5, dans laquelle le verre est utilisé en tant que matériau de correction pour des suprastructures dentaires métallo-céramiques ou en céramique pleine.

7. Utilisation selon la revendication 6, dans laquelle la suprastructure dentaire a la forme d'une couronne, d'un bridge ou d'une couronne partielle.

8. Produit dentaire façonné, qui présente une teneur en le verre selon l'une des revendications 1 à 5 et a la forme d'une couronne, d'une couronne partielle ou d'un bridge.
